# EUROPÄISCHE PATENTSCHRIFT

(11) **EP 2 624 766 B1**
(45) Veröffentlichungstag und Bekanntmachung des Hinweises auf die Patenterteilung: **25.02.2015**
(21) Anmeldenummer: 11767170.1
(22) Anmeldetag: 06.10.2011
(51) Int. Cl.: A61B 17/02

(54) **BELEUCHTBARER MEDIZINISCHER HALTEHAKEN**
ILLUMINATABLE MEDICAL RETAINING HOOK
CROCHET DE MAINTIEN MÉDICAL AVEC ÉCLAIRAGE

(30) Priorität: 07.10.2010 DE 102010047835
(43) Veröffentlichungstag der Anmeldung: 14.08.2013
(73) Patentinhaber: Heitland, Martina, 81929 München (DE); Gerg, Blasius, 83104 Hohenthann (DE)
(72) Erfinder: Heitland, Martina, 81929 München (DE); Gerg, Blasius, 83104 Hohenthann (DE)
(74) Vertreter: Müller - Hoffmann & Partner
(86) Internationale Anmeldenummer: PCT/EP2011/004995
(87) Internationale Veröffentlichungsnummer: WO 2012/045459

(56) Entgegenhaltungen:
- US-A- 3 638 644
- US-A1- 2007 066 872
- US-A1- 2007 208 226
- US-A1- 2009 069 634
- US-A1- 2009 112 068
- US-B2- 6 893 394
- US-B2- 7 306 559

## Beschreibung

Die Erfindung betrifft einen medizinischen Haltehaken zur Gewebespreizung bei chirurgischen Eingriffen mit einem an einem Handgriff angesetzten Hakenelement, das auch als chirurgischer Retraktor bezeichnet wird.

Bei chirurgischen Eingriffen, z.B. im Bauch- oder Thoraxbereich, ist es unabdingbar notwendig, das umgebende, beispielsweise oberhalb einer Operationsstelle liegende, Gewebe zurück-, d.h. aufgespreizt, zu halten. Dazu werden üblicherweise vom Hilfspersonal, beispielsweise einer Operationsschwester oder einem Assistenzarzt zu haltende, gegebenenfalls auch an einer seitlichen Haltevorrichtung zu befestigende, Haltehaken verwendet, die im Wesentlichen aus einem etwa rechtwinkligen Hakenelement aus flachem Edelstahlmaterial bestehen, das am halteseitigen Ende in eine Handgriff-Verdickung übergeht und in der Regel am freien, der Operationsstelle zugekehrten, Ende mit einer stumpfen Gewebe-Haltenase versehen ist.

Für den operierenden Chirurgen ist eine gute Ausleuchtung der Operationsstelle von zentraler Bedeutung. Die üblichen verstell- und einstellbaren Überkopfleuchten an Operationstischen erfüllen die Anforderungen an eine nach Möglichkeit In-situ-Beleuchtung bzw. -ausleuchtung der Operationsstelle oft nicht, da nicht selten zwei oder drei "Beobachtungsköpfe" zwischen der Überkopfleuchte und der Operationsstelle eine Abschattung verursachen.

Es ist bekannt, entlang der Oberseite der in Rede stehenden medizinischen Haltehaken innerhalb eines Metallröhrchens eine Lichtleitfaser zu führen mit einer Lichtaustrittsfläche am oder in der Nähe des freien Endes des Gewebe-Haltehakens. Diese Lichtleitfaser ist meist über ein geeignetes Kupplungselement und dann weiterführend über ein Glasfaserkabel an eine Kaltlichtquelle angeschlossen, deren Speisegerät sich in der Nähe des Operationstisches befindet und ihrerseits über ein Netzstromkabel gespeist wird. Diese Art der Beleuchtung einer Operationsstelle über den Haltehaken ist insofern unbefriedigend, als zu der ohnehin vorhandenen Vielzahl von Schläuchen und Kabeln an einem Operationstisch eine oder meist mehrere zusätzliche Kabelverbindungen für die In-situ-Ausleuchtung der Operationsstelle erforderlich sind. Aus der Druckschrift US 7 306 559 B2 sind ebenfalls verschiedene Ausführungsvarianten von chirurgischen Haltehaken (chirurgischen Retraktoren) bekannt, bei denen eine an einem Handgriff fixierbare, etwa rechtwinklig abgebogene, metallische Rückhalteklinge zusammen mit einer schichtartig darüber angeordneten Lichtleiterleiste in einem Handgriff fixiert sind, in dessen Gehäuse eine Stromquelle (Batterie, Akku) eingesetzt sein kann, die Licht in den Lichtleiter einspeist. Die Lichtleiterleiste ist im Bereich des freien Rückhalteabschnitts der Rückhalteklinge als Lichtemitter, z.B. in Form mehrerer Lampen, gestaltet, so dass das vom Haken erfasste Gewebe (Ader, Sehne, Knochen etc.) erkannt werden kann, nicht jedoch eine ausreichende Ausleuchtung der unmittelbaren Operationsstelle gewährleistet ist.

Aus dieser unbefriedigenden Situation ergibt sich die der Erfindung zugrunde liegende Aufgabe, nämlich ein autarkes medizinisches Hakenelement zur Gewebespreizung zu schaffen, mit dem sich auch bei einer längeren Operationsdauer ohne zusätzliche Wärmeabstrahlung eine gute örtliche Ausleuchtung der Operationsstelle bei einem chirurgischen Eingriff gewährleisten lässt.

Die Erfindung ist bei einem medizinischen Haltehaken zur Gewebespreizung bei chirurgischen Eingriffen mit einem an einen Handgriff ansetzbaren Hakenelement, bei dem der Handgriff als Hohlkörper zur Aufnahme eines elektrischen Energiespeichers gestaltet ist, und bei dem das dem Hakenelement zugekehrte Frontende des Handgriffs eine Lichtquelle nebst Energiequelle und Speiseschaltung eingesetzt ist, deren emittiertes Licht in eine frontendseitige Einstrahlfläche eines entlang des Hakenelements geführten Lichtleiters abgestrahlt und bis zu dessen freiem Ende geleitet wird, erfindungsgemäß dadurch gekennzeichnet, dass das gleichzeitig als Lichtleiter dienende Hakenelement aus einem lichtleitfähigen Polykarbonat- oder Polymethylmethacrylat-Material . mit guter Wärmeformbeständigkeit und optischer Klarheit, hoher mechanischer Belastbarkeit sowie Schlagzähigkeit besteht. Um eine konzentrierte Lichtführung zu gewährleisten, die es insbesondere erlaubt. mit Weißlicht-LEDs vergleichsweise geringer Leistung bei sehr guter Punktlichtausleuchtung der Operationsstelle auszukommen, ist erfindungsgemäß auf der der endseitigen Krümmung des Hakenelements abgekehrten Außenseite des Hakens, in dessen Längsrichtung verlaufend, ein Lichtführungssteg vorgesehen, mit einem sich verjüngenden, spitz zulaufenden, freien Ende, an dem das Punktlicht austritt. Dieser Lichtführungssteg weist die Form eines einstückig angeformten Griffels auf, mit rundem oder zumindest auf der Außenseite abgerundetem Querschnitt.

Im Sinne der Erfindung besteht die Lichtquelle ganz vorzugsweise aus einer licht-emittierenden Diode (LED), die ebenfalls vorzugsweise Weißlicht oder weitgehend weißes Licht abstrahlt. Als Lichtquelle kann auch ein RGB-Laser (Weißlichtlaser) in Frage kommen.

Es kann vorteilhaft sein, zwischen der Lichtemissionsfläche der Lichtquelle, insbesondere der Weißlicht-LED und dem Einstrahlende des Hakenelements, eine Linse einzusetzen, die insbesondere in das flache Einstrahlende des Hakenelements integriert sein kann. Um eine gute Punktlichtausleuchtung der Operationsstelle sicher zu stellen, kann es auch vorteilhaft sein, am freien Lichtaustrittsende oder -bereich eine Linse, insbesondere eine Sammellinse, vorzusehen, die beispielsweise auf den vorzugsweise schon den Lichtstrom verengend ausgeführten Endabschnitt des Lichtleiters aufgeklebt oder einstückig angeformt sein kann.

Das Hakenelement selbst weicht in seiner äußeren Gestaltung nicht grundsätzlich von den bisher bekannten Hakenelementen ab, die sich in der Praxis bewährt haben. Es wird in einem hochwertigen Spritzverfahren, das extrem glatte Oberflächen gewährleistet, aus einem Polykarbonatmaterial oder Polymethylmethacrylatmaterial (PMMA) mit guter Wärmeformbeständigkeit und optischer Klarheit hergestellt und zwar einstückig einschließlich des handgriffseitigen verdickten Anschlussteils beispielsweise mit Innenseitiger Quernut für eine Bajonettverbindung mit dem Handgriff. Das Hakenelement weist vorzugsweise in seiner Längsrichtung verlaufende gekrümmte Kanten und eventuell eine angefaste Lichtreflektlonsfläche im Bereich der Hauptkrümmung des Hakenelements auf. An seinem freien Ende ist eine quer zur Erstreckung der Haltenase liegende Lichtaustrittsfläche vorgesehen. Sofern der Haltehaken, was die Regel ist, die übliche 90°-Abbiegung aufweist, kann die an der Krümmungsaußenseite liegende angefaste Fläche in einem Winkel von 45° zu den beiden Achsen der Schenkel des Hakenelements stehen. Der Abbiegungs- oder Krümmungswinkel kann - wie an sich bekannt - auch deutlich größer oder kleiner als 90° sein. Üblicherweise kommen Haltehaken mit einem Abbiege- oder Krümmungswinkel α von 60°≤ α ≤120° zur Anwendung.

Das Hakenelement selbst weist vorteilhafter Weise einen flach-ovalen Querschnitt, einen flach-rechteckigen Querschnitt mit abgerundeten Kanten oder einen in dessen Längsrichtung verlaufenden konkaven Querschnitt, also die Form eines lang gestreckten, abgewinkelten Löffels, auf. Dadurch lässt sich zusammen mit der Auswahl des geeigneten PMMA- oder Polykarbonatmaterials eine gute Biegesteifigkeit bzw. eine Zugkraft im Bereich der Gewebehaltenase von 100 bis zu einigen hundert N erreichen.

Das bereits erprobte Polykarbonatmaterial bzw. das erprobte Polymethylmethacrylat-Material mit sehr glatten äußeren Oberflächen am Hakenelement ermöglichen eine gute Lichtführung von der Emissionsfläche der Lichtquelle, insbesondere der Weißlicht-LED bis zur Lichtaustrittsfläche an der Gewebehaltenase. Damit lässt sich trotz der vergleichsweise sehr geringen Leistung der Lichtquelle eine gute In-situ-Ausleuchtung der Operationsstelle gewährleisten. Der Handgriff, an den das Hakenelement beispielsweise über eine formschlüssige Kupplung, den schon erwähnten Bajonettverschluss, einen Schraubverschluss, einen Steckverschluss oder auch einen Ringschnappverschluss angeflanscht bzw. angesetzt wird, ist als Hohlkörper gestaltet, der einen Energiespeicher, insbesondere eine Batterie oder einen Akkumulator, aufnimmt. Zwischen dem dem Hakenelement zugekehrten Ende und dem Energiespeicher im Inneren ist die Versorgungsplatine mit Speiseschaltung und Lichtquelle eingesetzt. Bei erprobten Modellen des Erfindungsgegenstands ist der Handgriff oder das Handstück unterseitig mit einem abnehmbaren Deckel, beispielsweise einem Deckel mit Gewinde oder Schnappverschluss, versehen, der vorteilhafter Weise durch weiteres Verdrehen gleichzeitig einen Schalter zur Ein-/Ausschaltung der Lichtquelle betätigt.

Bei einer derzeit bevorzugten, erprobten Ausführungsvariante der Erfindung ist der Anschlussabschnitt des Hakenelements zum Handgriff als eine auf die Lichtaustrittsseite des Handgriffs formschlüssig aufsteckbare Kappe ausgebildet, an deren geschlossener Stirnseite, dem Kappenboden, eine Schaltkulisse eingeformt ist. durch die sich beim Verdrehen des Haltehakens relativ zum Gehäuse des Handgriffs ein auf der oberen Stirnfläche des Handgriffgehäuses vorspringender, federvorbelasteter Schaltknopf zur Ein-/Ausschaltung der Lichtquelle betätigen lässt.

Das Hakenelement lässt sich einschließlich des angeformten Lichtführungsstegs in einer hoch polierten Spritzform aus dem erwähnten Kunststoffmaterial in einem einzigen Arbeitsgang herstellen. Die LED-Weißlichtquelle sollte einen Lichtstrom im Bereich von 601m ≤ Φᵥ ≤ 200lm bei einer für Batterie-/Akkubetrieb geeigneten Stromaufnahme von 100 mA ≤ I_{F} ≤ 1A liefern. Geeignete LEDs sind beispielsweise die zur Zeit von der Firma OSRAM Opto Semiconductors angebotenen Typen OSLON SSL, LCW CQ7P und LUW CQ7P.

Ausführungsbeispiele der Erfindung, die bereits erprobten Prototypen im Wesentlichen entsprechen, werden nachfolgend anhand der Zeichnung erläutert.
**Figur 1** zeigt einen beleuchtbaren medizinischen Haltehaken zur Gewebespreizung bei chirurgischen Eingriffen nicht gemäß der Erfindung.
**Figur 2** veranschaulicht mit
   - Teilfigur 2A die isometrische Ansicht einer Ausführungsform des beleuchtbaren Hakenelements eines medizinischen Retraktors mit erfindungsgemäßen Merkmalen;
   - Teilfigur 2B die Draufsicht auf das Hakenelement nach Teilfigur 2A mit darunter gezeichneter Schnittdarstellung, gesehen in Richtung der Pfeile am Schnitt B-B in Fig. 2B;
   - Teilfigur 2C die Seitenansicht des Hakenelements nach Fig. 2A, und
   - Teilfigur 2D die Längsschnittdarstellung des Hakenelements nach Fig. 2A, gesehen in Richtung der Pfeile A-A auf den Längsschnitt in Fig. 2B.

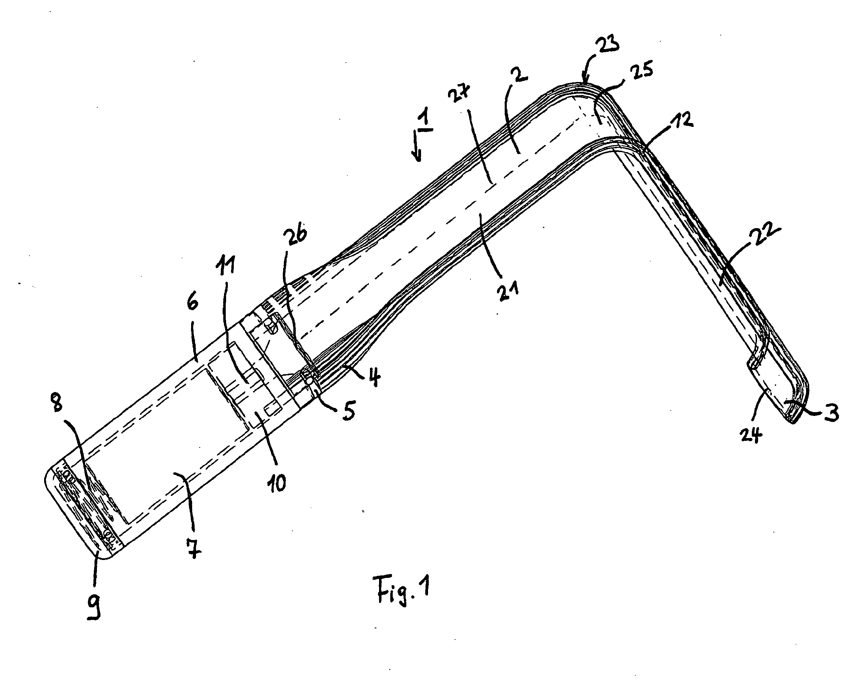

Der medizinische Haltehaken 1 nach Fig. 1 besteht hinsichtlich seiner Haltefunktion aus einem einstückigen zweischenkligen Hakenelement 2, dessen griffseitiger Schenkel 21 und dessen freier Schenkel 22 über eine Krümmung 23 von im dargestellten Beispiel 90° oder im Wesentlichen 90° einstückig verbunden sind. Der Krümmungswinkel kann auch größer oder kleiner als 90° sein, also etwa im Bereich von 60° s α ≤ 120°, gewählt sein. In der dargestellten Ausführungsvariante weist das Hakenelement 1 eine nach innen bzw. unten gerichtete konkave Krümmung 12 auf also die Form eines lang gestreckten Löffels, wobei die freien Kanten selbstverständlich abgerundet sind. An seinem unteren freien Ende weist das Hakenelement 2 eine nach innen in Richtung des Handgriffs abgebogene Gewebe-Haltenase 3 auf, deren freie Endfläche 24 die Lichtaustrittsfläche bildet. Der Querschnitt des Hakenelements 2 ist nicht auf die dargestellte Form eines lang gestreckten Löffels beschränkt. Die Querschnittsform kann auch lang-oval oder rechteckförmig mit abgerundeten Kanten sein. Im Bereich der Krümmung 23 kann, insbesondere wenn ein ovaler Querschnitt gewählt wird, außen eine angefaste Fläche 25 vorhanden sein, die bei 90° Biegung des Hakenelements 2 in einem Winkel von 45° zu den jeweiligen Erstreckungsrichtungen der beiden Schenkel 21, 22 steht und zusätzlich eine gute Lichtumlenkung gewährleistet.

Im Bereich des Handgriffs 6 ist das Hakenelement 2 mit einer Aufweitung 4 versehen, die in einem im Wesentlichen kreisförmigen Querschnitt endet, dessen Außendurchmesser auf den Durchmesser des Handgriffs 6 angepasst ist. Der Handgriff 6 ist beispielsweise als metallische Hülse beispielsweise aus Edelstahl gestaltet, an deren dem Hakenelement 2 zugekehrtem Ende die Versorgungsplatine 10 mit Versorgungsschaltung für eine weißlichtemittierende LED als Lichtquelle 11 eingesetzt ist. Eine formschlüssige Kupplung 5, beispielsweise nach Art eines Bajonettverschlusses, verbindet das Hakenelement 2 mit dem Handgriff 6. Die Lichtquelle 11 kann von einer Keramikfassung umgeben sein bzw. die zugehörige Versorgungsplatine 10 kann aus Keramik hergestellt sein, so dass eine ausreichende Wärmeverteilung bzw. -abführung gewährleistet ist, insbesondere wenn die LED-Fassung bzw. die Platine noch mit einer Aluminiummanschette versehen ist. Der hülsenartige Handgriff 6 dient im Wesentlichen zur Aufnahme eines elektrischen Energiespeichers 7, beispielsweise einer handelsüblichen Batterie oder eines Akkumulators. Unterseitig also im Bereich des freien Endes des Handgriffs 6 ist innenseitig eine Druckfeder 8 eingesetzt, die den elektrischen Energiespeicher 7 gegen den entsprechenden Anschlusspol der Platine 10 bzw. der Versorgungsschaltung für die LED 11 drückt. Am freien Ende ist die Hülse des Handgriffs 6 mit einem aufschraubbaren Deckel 9 oder Klemmdeckel abgeschlossen, in den vorteilhafter Weise (nicht dargestellt) ein Schalterelement integriert ist, so dass sich beim Verdrehen bzw. Weiterdrehen des Deckels 9 in Verschlussrichtung die LED 11 aktivieren lässt.

Das von der Lichtquelle 11 emittierte Weißlicht wird in die anschlussseitige Endfläche 26 des Hakenelements 2 eingestrahlt und innerhalb des Kunststoffmaterials des Hakenelements 2 geführt; es tritt dann an der Lichtaustrittsfläche 24 der Gewebe-Haltenase 3 wieder aus und gewährleistet so eine gute Ausleuchtung der Operationsstelle. Um eine möglichst verlustfreie Führung des von der Lichtquelle 11 emittierten Lichts zu gewährleisten, ist es von Vorteil, die Lichteintrittsfläche 26 am Hakenelement möglichst nah an die Emissionsfläche der LED heranzurücken. Es kann auch von Vorteil sein, entweder an der Lichtaustrittsfläche der LED oder an der Lichteintrittsfläche 26 des Hakenelements 2 eine integrierte Linse (nicht dargestellt) vorzusehen.

Eine nicht erfindungsgemäße Ausführungsvariante besteht darin, in das Material des Hakenelements 2 eine Lichtleitfaser 27 (gestrichelt angedeutet) integriert einzubringen um insbesondere bei einer dann möglichen Verringerung der Leistung der Lichtquelle dennoch eine optimale Ausleuchtung der Operationsstelle zu gewährleisten.

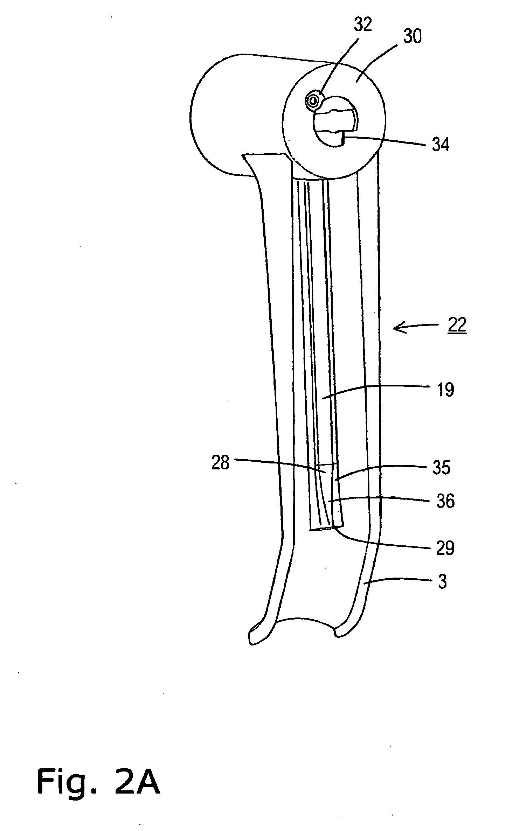

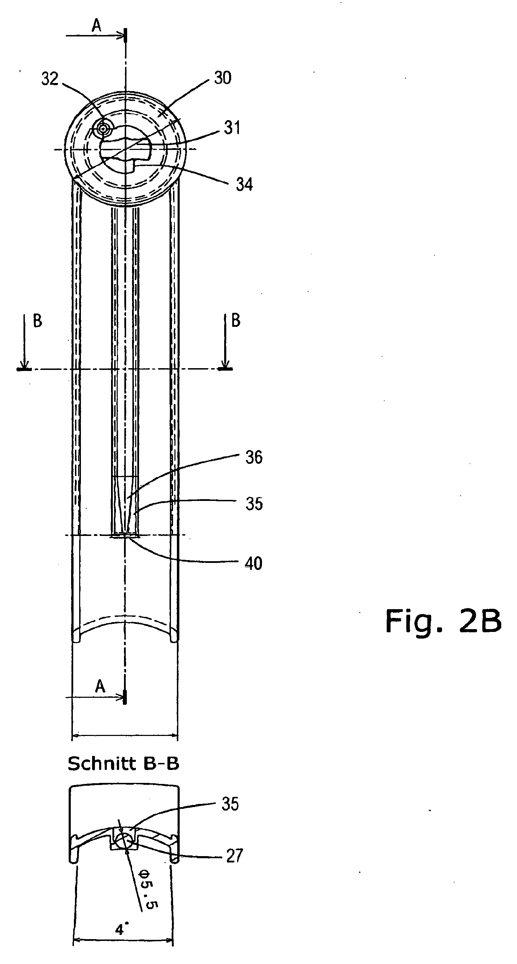

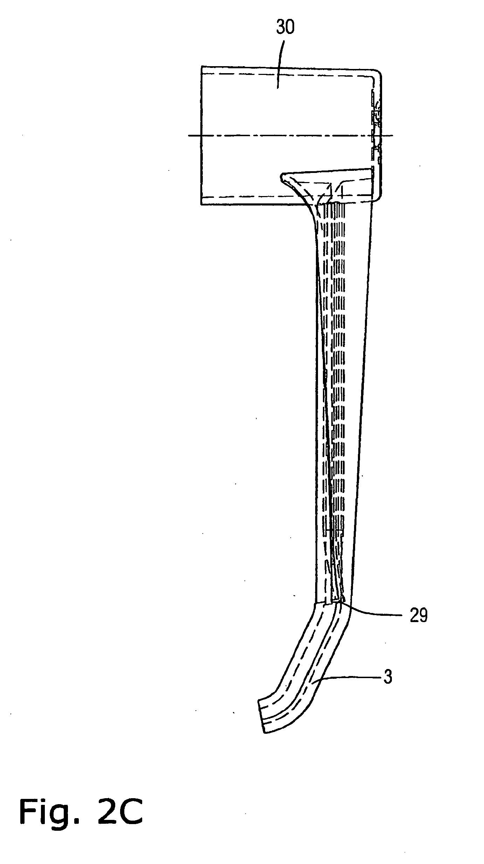

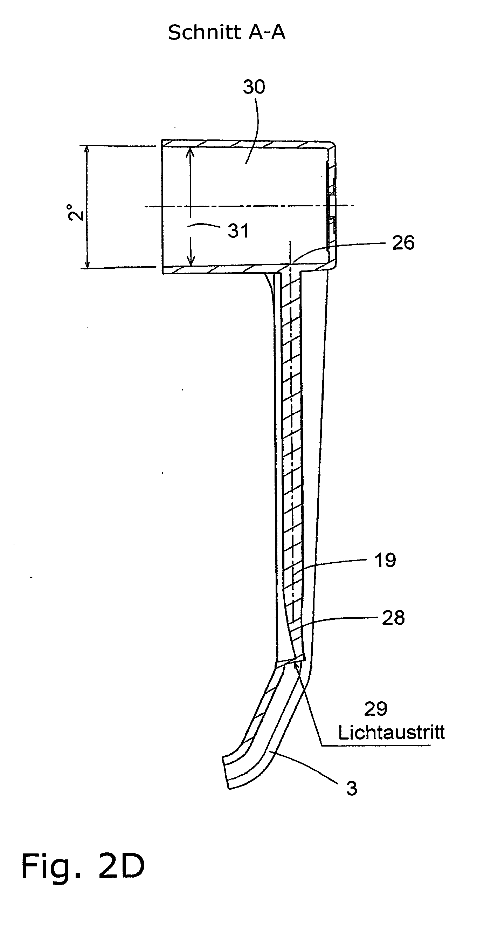

Bei dem derzeit bevorzugten Ausführungsbeispiel der Erfindung gemäß Fig. 2 mit Teilfiguren 2A bis 2D besteht das Hakenelement 2 im Wesentlichen aus einer Kappe 30, die entsprechend formschlüssig angepasst auf den Handgriff 6 aufgesteckt wird, wobei ein geringer Innenkonus von beispielsweise 2° (vgl. Fig. 2D) das formschlüssige Aufschieben auf das lichtseitige Ende des Handgriffs erleichtert und dabei einen guten, weitgehend spielfreien Sitz der Kappe 30 auf dem Handgriff 6 gewährleistet. Um eine positionsrichtige Ausrichtung der Lichtquelle 11 auf das nachfolgend zu beschreibende Hakenelement 2 mit integriertem zusätzlichem Lichtleiter zu gewährleisten, ist auf der lichtseitigen Stirnfläche des Handgriffs 6 ein beispielsweise schwalbenschwanzförmiger Ansatz ausgebildet, dem eine entsprechende Aussparung 31 im Boden der Kappe 30 entspricht, so dass die Kappe nur in einer gewünschten Drehwinkelposition auf den Handgriff 6 aufgesetzt werden kann und dann anschließend aufgrund eines radialen Anschlags 34 nur in einem Winkelbereich von beispielsweise 90° verdreht werden kann aus Gründen, welche weiter unten näher erläutert werden.

Am Außenmantel der Kappe 30 ist der in einem Winkel von 75° ≤ α ≤ 105°, vorzugsweise mit einem Winkel von 90° zur Kappe 30 abstehende freie Schenkel 22 des Hakenelements 2 einstückig angesetzt, der - ähnlich wie in Fig. 1 - die Form eines lang gestreckten Löffels aufweist mit in Richtung des Handgriffs abgewinkelter Haltenase 3.

Um eine gegenüber Fig. 1 verbesserte konzentrierte Lichtführung zu gewährleisten, ist am Löffelboden des Schenkels 22 eine mittig in Längserstreckung des Schenkels 22 verlaufende Senke oder Nut 35 eingeformt, die zur Halterung und Führung eines Lichtführungsstrangs 19 von der Lichtquelle 11 zur freien Spitze 36, dem Lichtaustrittspunkt, dient, wobei das vordere freie Ende des Lichtführungsstrangs 19 als konisch verjüngte Spitze 28 ausgeführt ist. Die Figs. 2A, 2B und 2C lassen gut erkennen, dass der Lichtaustritt über eine sehr geringe, fast punktförmige Lichtauskoppelfläche 36 erfolgt. Um eine noch schärfere Bündelung des von der Lichtquelle 11 in den Schenkel 22 des Haltehakens 2 eingestrahlten Lichts zu erreichen, kann der griffelartige Lichtführungsstrang 19 an seiner endseitigen punktartigen Lichtauskoppelfläche 36 mit einer in Fig. 2B angedeuteten Linse 40, insbesondere einer Sammellinse, versehen sein, über die die Lichtauskoppelung erfolgt, Das Licht tritt dann praktisch als punktförmiger Lichtstrahl aus, so dass eine erwünschte punktgenaue Ausleuchtung der Operationsstelle optimal verwirklicht ist.

Die Kappe 30 bildet zusammen mit dem Schenkel 22 den Haltehaken, der einschließlich des in der Nut 35 geführten Lichtführungsstrangs 19 einstückig in einer hoch polierten Spritzgussform aus dem erwähnten gut lichtleitfähigen Polykarbonat- oder Polymethylmethacrylat-Material mit hoher optischer Klarheit und mechanischer Belastbarkeit hergestellt wird.

Wie oben erläutert, lässt sich der Haltehaken, im Wesentlichen bestehend aus der Kappe 30 und dem Schenkel 22 mit Lichtführungsstrang 19, in einem bestimmten Winkel, beispielsweise von 90°, gegen den Handgriff 6 verdrehen, bzw. verschwenken. Durch diese eingeschränkte Relativdrehbewegung zwischen dem Haltehaken und dem Handgriff lässt sich ein Ein-/Ausschalter für die Lichtquelle 11, bzw. die Stromspeiseschaltung der Lichtquelle 11, betätigen. Zu diesem Zweck kann auf der dem Hakenelement 2 zugewandte Stirnseite des Handgriffs ein federvorbelasteter Druckschalter (nicht dargestellt) mit abgerundetem Kopf vorgesehen sein, der in einer am inneren Boden der Kappe 30 ausgesparten Kulissen-Nut oder trichterartigen Schräge 32 einrastend geführt wird. Sobald das Hakenelement 2 beim Verdrehen die richtige Ausrichtposition zwischen Lichtquelle und dem Einstrahlende des Lichtführungsstrangs 19 erreicht hat, springt der federvorbelastete Kopf in eine lochartige Vertiefung, vorzugsweise ein Sackloch im innenseitigen Bodenbereich der Kappe 30, wodurch die Lichtquelle 11 einschaltet. Durch diese Besonderheit der Konstruktion werden, insbesondere im Bereich des Handgriffs 6 keine freiliegenden Abschnitte, Verbindungsnuten und dergleichen mehr benötigt, was für die Sterilisation und Keimfreiheit des Handgriffs von besonderem Vorteil ist. Das Hakenelement 2 selbst mit Kappe 30 und Schenkel 22 einschließlich Lichtführungsstrang 19 wird als vorsterilisierter Einwegartikel bereitgestellt.

Im täglichen Routinebetrieb eines Krankenhauses ist der Arbeits- und Kostenaufwand unter anderem für die Gerätesterilisation im chirurgischen Bereich ein erheblicher Kostenfaktor. Hier leistet die Erfindung einen Beitrag zur Kostenreduzierung. Bei einem chirurgischen Eingriff in Körper-Hohlräumen (Bauch, Thorax), in der plastischen Chirurgie oder auch in der Zahnmedizin werden, natürlich in unterschiedlichen Größen, regelmäßig pro Operationstermin mehrere Haltehaken der hier in Rede stehenden Art benötigt, die bisher anschließend zerlegt, gereinigt und sterilisiert werden müssen. Mit der Erfindung ergibt sich nicht nur der Vorteil eines kabellos und problemlos verwendbaren medizinischen Haltehakens sondern auch die vorteilhafte Möglichkeit, das auf den Handgriff mit Energiespeicher aufsetzbare Hakenelement - wie erwähnt - als vorsterilisierten Einwegartikel einzusetzen, der nach einem chirurgischen Eingriff weder gereinigt werden muss noch sterilisiert zu werden braucht. Bei der derzeit in Erprobung befindlichen Ausführungsform der Erfindung ist der Handgriff noch als hülsenartiges Element aus Edelstahl gefertigt. Es ist jedoch auch möglich, den Handgriff als Kunststoff-Spritzteil zu fertigen, so dass eine vollständige Entsorgung des gesamten Hakenelements 2 nach einem chirurgischen Eingriff möglich wird. Zu denken ist auch an eine einstückige Fertigung des Haltehakens einschließlich Handgriff, wobei die Bestückung mit der LED-Lichtquelle 11 einschließlich Versorgungsplatine und elektrischer Energiequelle von der Unterseite, d.h. von der Seite des Deckels 9, erfolgen kann. In diesem Fall kann auch an eine elektrische Energiequelle geringerer Leistung gedacht werden, die als einziges Element nach einem Gebrauch separat entsorgt werden müsste.

## Patentansprüche

1. Autark beleuchtbarer medizinischer Haltehaken zur Gewebespreizung bei chirurgischen Eingriffen mit einem Handgriff und einem daran ansetzbaren Hakenelement (2), bei dem der Handgriff (6) als Hohlkörper zur Aufnahme eines elektrischen Energiespeichers (7) gestaltet ist, und bei dem in das dem Hakenelement zugekehrte Frontende des Handgriffs (6) eine Lichtquelle (11) nebst Energiequelle und Speiseschaltung eingesetzt sind, deren emittiertes Licht in eine frontendseitige Einstrahlfläche (26) eines entlang des Hakenelements geführten Lichtleiters eingestrahlt und bis zu dessen freiem Ende geleitet wird, **dadurch gekennzeichnet, dass**
das gleichzeitig als Lichtleiter dienende Hakenelement (2) aus einem lichtleitfähigen Polykarbonat- oder Polymethylmethacrylatmaterial mit guter Wärmeformbeständigkeit und optischer Klarheit, hoher mechanischer Belastbarkeit sowie Schlagzähigkeit besteht,
entlang einer Außenfläche des Hakenelements ein Lichtführungssteg (27) mit gekrümmtem Querschnitt angebracht ist, der einen Lichtführungsstrang von der Lichtquelle (11) bis in die Nähe des freien Endes des Hakenelements bildet und im Bereich seines freien Lichtaustrittsendes (28) konisch spitz zulaufend mit verjüngter Lichtauskoppelfläche (29) ausgebildet ist, die einen punktähnlichen Lichtaustritt gewährleistet, und dass
der Lichtführungssteg aus dem gleichen lichtleitfähigen Material und einstükkig mit dem Hakenelement verbunden hergestellt ist.

2. Medizinischer Haltehaken nach Anspruch 1, **dadurch gekennzeichnet, dass** das Hakenelement (2) über eine formschlüssige Kupplung (5) mit dem zugeordneten Frontende des Handgriffs verbunden ist.

3. Haltehaken nach Anspruch 1, **dadurch gekennzeichnet, dass** die Lichtquelle eine lichtemittierende Diode ist.

4. Haltehaken nach Anspruch 3, **dadurch gekennzeichnet, dass** die lichtemittierende Diode (LED) eine weißlichtemittierende LED ist.

5. Haltehaken nach Anspruch 1, **dadurch gekennzeichnet, dass** zwischen der Lichtemissionsfläche und der Einstrahlfläche des Hakenelements eine Linse eingesetzt ist.

6. Haltehaken nach Anspruch 1, **dadurch gekennzeichnet, dass** der Handgriff zur Auswechslung des elektrischen Energiespeichers an seinem freien Aussenende mit einem Deckel (9) mit integriertem Schalter zur Ein-/Ausschaltung der Lichtquelle versehen ist.

7. Haltehaken nach Anspruch 1, **dadurch gekennzeichnet, dass** der Lichtführungssteg einen runden Querschnitt aufweist.

8. Haltehaken nach Anspruch 7, **dadurch gekennzeichnet, dass** das Hakenelement einstückig mit einer Haltekappe (30) ausgebildet ist, deren Innendurchmesser (31) auf den lichtaustrittsseitigen Endbereich des Handgriffs (6) angepasst ist.

9. Haltehaken nach Anspruch 8, **dadurch gekennzeichnet, dass** die Haltekappe (30) bodeninnenseitig eine in Ausrichtung des Handgriffs ansteigend/abfallend verlaufende Kulissenspur (32) oder eine konische Vertiefung aufweist, in die bei auf dem Handgriff aufgesetztem Hakenelement ein federvorgespannter Schaltknopf an der von der Haltekappe (30) überdeckten Stirnfläche zur Ein-/Ausschaltung der Lichtquelle bei Relativverdrehung von Hakenelement und Handgriff einrastet.

10. Haltehaken nach Anspruch 7, **dadurch gekennzeichnet, dass** an der verjüngten Lichtaustrittsfläche (29) zusätzlich eine Linse (40) angeordnet ist.

## Claims

1. An autonomously illuminatable medical retaining hook for holding tissue open during surgical interventions, comprising a handle and a hook element (2), which can be attached thereto, in the case of which the handle (6) is designed as hollow body for accommodating an electrical energy storage means (7) and in the case of which a light source (11) together with energy source and supply circuit, the emitted light of which is irradiated into an irradiation surface (26) on the front end side of a light conductor, which is guided along the hook element, and which is guided to the free end thereof, is inserted into the front end of the handle (6), which faces the hook element, **characterized in that**
the hook element (2), which at the same time serves as light conductor, consists of a light-conducting polycarbonate or polymethylmethacrylate material having a good heat resistance and optical clarity, high mechanical load-bearing capacity as well as impact toughness
a light distribution line (27), comprising a curved cross section, which forms a light guide from the light source (11) into the vicinity of the free end of the hook element and which is embodied so as to taper conically comprising a narrowed light decoupling surface (29), which ensures a spot-like light emission, in the area of its free light emission end (28), is attached along an outer surface of the hook element, and **in that**
the light distribution line is made of the same light-conducting material and is made so as to be connected in one piece with the hook element.

2. The medical retaining hook according to claim 1, **characterized in that** the hook element (2) is connected to the assigned front end of the handle via a form-fit coupling (5).

3. The retaining hook according to claim 1, **characterized in that** the light source is a light emitting diode.

4. The retaining hook according to claim 3, **characterized in that** the light emitting diode (LED) is a white light emitting LED.

5. The retaining hook according to claim 1, **characterized in that** a lens is inserted between the light emission surface and the irradiation surface of the hook element.

6. The retaining hook according to claim 1, **characterized in that**, for exchanging the electrical storage device, the handle is provided on its free outer end with a cover (9) comprising an integrated switch for turning on/turning off the light source.

7. The retaining hook according to claim 1, **characterized in that** the light distribution line encompasses a round cross section.

8. The retaining hook according to claim 7, **characterized in that** the hook element is embodied in one piece with a retaining cap (30), the inner diameter of which (31) is adapted to the end area of the handle (6) on the light emission side.

9. The retaining hook according to claim 8, **characterized in that**, on the inner side of the bottom, the retaining cap (30) encompasses a connecting member track (32), which runs so as to rise/drop in orientation of the handle, or which encompasses a conical depression, with which a spring preloaded push button switch on the front surface, which is covered by the holding cap (30), engages for tuning on/turning off the light source in response to a relative twisting of hook element and handle when the hook element is attached to the handle.

10. The retaining hook according to claim 7, **characterized in that** a lens (40) is additionally arranged on the narrowed light emission surface (29).

## Revendications

1. Crochet de maintien médical à éclairage autonome, pour écarter les tissus lors d'interventions chirurgicales, avec une poignée et un élément à crochet (2) apte à être rattaché à celle-ci, étant précisé que ladite poignée (6) est conçue comme un corps creux destiné à recevoir un accumulateur d'énergie électrique (7), et qu'il est prévu, placée dans l'extrémité avant de la poignée (6) qui est tournée vers l'élément à crochet, une source de lumière (11), en plus de la source d'énergie et du circuit d'alimentation, dont la lumière émise est introduite dans une surface de rayonnement (26) située côté extrémité avant, le long d'un conducteur de lumière longeant l'élément à crochet, et est amenée jusqu'à l'extrémité libre dudit guide de lumière, **caractérisé**
**en ce que** l'élément à crochet (2) qui sert en même temps de conducteur de lumière, se compose d'un matériau en polycarbonate ou en poly(méthacrylate de méthyle) qui présente une bonne stabilité dimensionnelle à la chaleur et une clarté optique, une solidité mécanique et une résistance aux chocs élevées,
**en ce qu'**il est prévu, le long d'une surface extérieure de l'élément à crochet, une nervure de guide de lumière (27) à section transversale courbe, qui forme un tronçon de guide de la source de lumière (11) jusqu'à proximité de l'extrémité libre de l'élément à crochet, et qui, dans la zone de son extrémité de sortie de lumière libre (28), s'effile en pointe conique et comporte une surface de sortie de lumière (29) rétrécie qui garantit une sortie de lumière en forme de point, et
**en ce que** la nervure de guide de lumière est fabriquée à partir du même matériau conducteur de lumière que l'élément à crochet et est reliée d'une seule pièce à celui-ci.

2. Crochet de maintien médical selon la revendication 1, **caractérisé en ce que** l'élément à crochet (2) est relié par l'intermédiaire d'un accouplement par complémentarité de forme (5) à l'extrémité avant associée de la poignée.

3. Crochet de maintien selon la revendication 1, **caractérisé en ce que** la source de lumière est une diode émettrice de lumière.

4. Crochet de maintien selon la revendication 3, **caractérisé en ce que** la diode émettrice de lumière (LED) est une LED à lumière blanche.

5. Crochet de maintien selon la revendication 1, **caractérisé en ce qu'**une lentille est placée entre la surface d'émission de lumière et la surface d'introduction de l'élément à crochet.

6. Crochet de maintien selon la revendication 1, **caractérisé en ce que** la poignée, en vue du remplacement de l'accumulateur d'énergie électrique, est pourvue, à son extrémité extérieure libre, d'un couvercle (9) à commutateur intégré pour une mise en marche/arrêt de la source de lumière.

7. Crochet de maintien selon la revendication 1, **caractérisé en ce que** la nervure de guide de lumière présente une section transversale ronde.

8. Crochet de maintien selon la revendication 7, **caractérisé en ce que** l'élément à crochet est réalisé d'une seule pièce avec un couvercle de support (30) dont le diamètre intérieur (31) est adapté à la zone d'extrémité, située côté sortie de lumière, de la poignée (6).

9. Crochet de maintien selon la revendication 8, **caractérisé en ce que** le couvercle de support (30) comporte, du côté intérieur du fond, une coulisse (32) qui va en montant/descendant en direction de la poignée ou un creux conique dans lequel, quand l'élément à crochet est posé sur la poignée, un bouton de manoeuvre contraint par ressort s'enclenche sur la surface frontale couverte par le couvercle de support (30), pour mettre en marche/arrêter la source de lumière lors d'une rotation relative de l'élément à crochet et de la poignée.

10. Crochet de maintien selon la revendication 7, **caractérisé en ce qu'**une lentille (40) est disposée en supplément sur la surface de sortie de lumière effilée (29).
